# EUROPEAN PATENT SPECIFICATION

(11) **EP 0 598 462 B1**
(45) Date of publication and mention of the grant of the patent: **16.12.1998**
(21) Application number: 93250281.8
(22) Date of filing: 18.10.1993
(51) Int. Cl.: A61B 17/068

(54) **Surgical instrument for applying fasteners**
Chirurgisches Gerät zum Anbringen von Klammern
Instrument chirurgical pour poser les agrafes

(30) Priority: 28.10.1992 DE 4236995
(43) Date of publication of application: 25.05.1994
(73) Proprietor: ETHICON INC., Somerville, New Jersey 08876-0151 (US)
(72) Inventor: Bilotti, Federico, D-22559 Hamburg (DE); Wohlers, Udo, D-22397 Hamburg (DE)
(74) Representative: UEXKÜLL & STOLBERG

(56) References cited:
- EP-A- 0 507 605
- US-A- 4 496 090
- US-A- 4 595 007
- US-A- 4 724 840

## Description

The present invention relates to a surgical instrument for applying fasteners according to the preamble of claim 1.

Such an instrument is known from U.S. Patent No. 4,595,007. In the known instrument a plurality of unformed straight fasteners having pointed ends is provided to a hollow tube applying head through a spring-loaded magazine. The driver being actuated by an actuating element pushes fasteners down the hollow tube to the curved pointed open end of the hollow tube applying head. After placing the front fastener the driver is withdrawn to accept another straight fastener in the line. The instrument can be used for joining tissue together by means of the fasteners which are curved while they are pushed down the curved end portion of the hollow tube applying head and forced through the tissue. In this way annular structures are formed. If the end of the tip of the hollow tube applying head is slightly offset laterally from the longitudinal axis of the hollow tube applying head, the pointed end of the fastener will be offset or laterally spaced from the trailing end as the fastener is placed.

The disadvantage of the known surgical instrument is that in the magazine the fasteners are placed side by side in a linear arrangement. If the magazine is designed to house a larger number of fasteners which is necessary for many kinds of surgical operations, the magazine gets clumsy and the surgical instrument is not handy any more. In particular, it cannot be applied for endoscopic purposes because its size does not permit introduction, e.g., into the abdominal cavity through an ordinary trocar sleeve.

It is an object of the present invention to provide a surgical instrument for applying fasteners of the type described above which is easy to use and compact, while in its fastener magazine is space enough for a sufficient number of fasteners, and which is also applicable for endoscopic purposes.

This problem is solved by the surgical instrument for applying fasteners having the features of claim 1. Advantageous embodiments are described in the dependent claims.

The fastener magazine of the surgical instrument according to the present invention comprises a rotatable drum which is biased by a spring so that it rotates in a given direction unless it is blocked, e.g. by the fastener to be applied. The fasteners, which are straight before they are applied, are arranged along the periphery of the drum, their axes being essentially parallel to the rotational axis of the drum. Because of this magazine design it is possible to incorporate the fastener magazine into the shaft of the surgical instrument so that there are no laterally protruding magazine portions. The shaft including the fastener magazine can be made small enough for use in endoscopic applications when the distal portion of the shaft including the fastener magazine and the fastener tube are inserted into, e.g., the abdominal cavity through a trocar sleeve.

In a preferred embodiment the fastener tube extends in proximal direction into the fastener magazine and has a longitudinal opening in this region through which the fastener to be applied is moved into the fastener tube. The wall of the fastener tube opposing this longitudinal opening forms a stop which prevents the drum from rotating. Preferably the drum comprises trough-like recesses on its lateral area for holding the fasteners. In this way the driving force of the spring is transmitted from the drum to the fasteners. The fastener next to the fastener to be applied is pressed against the fastener to be applied which already lies in the fastener tube and which is pressed against the fastener tube wall portion forming the stop. In order to apply the fastener to be applied the driving means is moved in distal direction by means of the actuating member and pushes the fastener to be applied down the fastener tube where it is bent and finally pushed out from the surgical instrument. As long as the distal end portion of the driving means occupies the place of the fastener to be applied inside the fastener magazine, rotational movement of the drum is still blocked. When the driving means is retracted, however, the drum rotates under the action of the spring and moves the next fastener into the fastener tube until it is stopped by the fastener tube wall portion opposite to the longitudinal opening.

Preferably the distal end portion of the driving means is flexible, e.g. by fixing a helical spring to the distal end of a rod serving as driving means. When the driving means is actuated the helical spring can adjust to the curved shape of the distal end portion of the fastener tube so that it is able to push out the fastener to be applied completely.

In a preferred embodiment the distal portion of the driving means, the fastener magazine and the fastener tube are included in a magazine member which is detachably fixed to the shaft, e.g. by means of a bayonet joint allowing for the coupling of the distal portion of the driving means to the proximal portion of the driving means. The magazine member is usually contaminated during an operation. It is preferably designed as a disposable article which can be easily removed from the shaft of the surgical instrument when it is coupled by means of a bayonet joint. The other parts of the surgical instrument can be used again after sterilization. - Alternatively, the whole surgical instrument may be designed to be fully disposable.

In the following the present invention is described in more detail by means of an embodiment. The drawings show:
- Figure 1: a longitudinal section of the surgical instrument for applying fasteners according to the present invention, the magazine member in the distal portion of the shaft being removed;
- Figure 2: a longitudinal section of the magazine member of the surgical instrument for applying fasteners according to the present invention, the scale being larger than in Figure 1;
- Figure 3: an enlarged side view of the fastener tube shown in Figure 2;
- Figure 4: a cross section through the fastener tube of Figure 3 along the line A-A;
- Figure 5: an enlarged longitudinal section of the magazine shown in Figure 2;
- Figure 6: a projectional view along the arrow X of Figure 5; and
- Figure 7: a cross section through the magazine of Figure 5 along the line B-B.

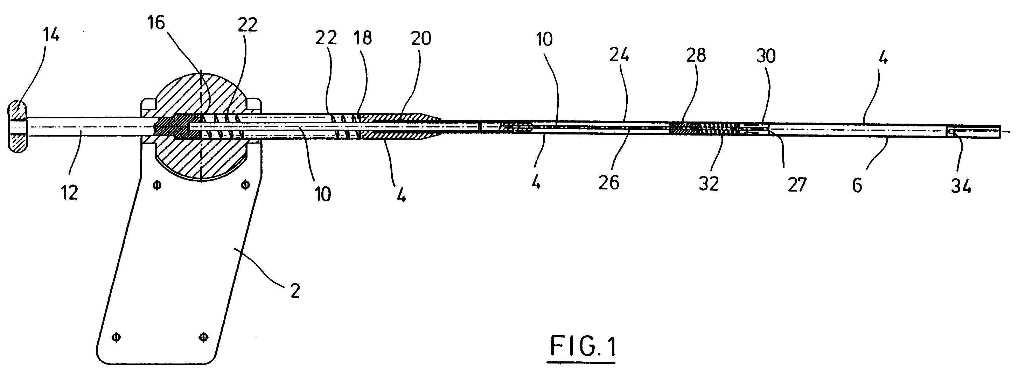

Figure 1 shows an embodiment of the surgical instrument according to the present invention, a magazine member being removed from the distal end. In the proximal region the instrument comprises a handle 2. A shaft 4 is attached to the handle 2. In the proximal region the diameter of the shaft 4 is larger than in the distal region in order to increase rigidity of the surgical instrument. In the distal region the diameter of the shaft 4 is small enough for inserting the instrument through an ordinary (e.g. 5 mm inner diameter) or large trocar sleeve into e.g. the abdominal cavity. The distal portion of the shaft 4 is formed by a sleeve 6 which is able to accommodate a magazine member which will be described below.

Inside the shaft 4 an essentially rod-like driving means 10 is guided in a longitudinally movable manner. The proximal end of the driving means 10 is fixed to an actuating member 12 which is slidably mounted in the handle 2. A button 14 is attached to the proximal end of the actuating member 12. The surgeon can easily press the button 14 with his thumb when he holds the handle 2 in his hands. Between an abutting surface 16 formed at the distal end of the actuating member 12 and an abutting surface 18 formed at the proximal end of a connection member 20 is situated a compression spring 22 which is biased to press the actuating member 12 and the driving means 10 in proximal direction.

A middle portion 24 of the shaft 4 having a smaller diameter than the proximal portion of the shaft 4 is attached to the connection member 20. In the middle portion 24 of the shaft 4 the diameter of the driving means 10 is changed. The middle portion 26 of driving means 10 is shaped as a thin cylindrical rod, whose distal end 27 is close to the end of the middle portion 24 of shaft 4. The distal portion of shaft 4 is formed as a sleeve 6 which has the same outer diameter as the middle portion 24 of shaft 4. In its distal end region the middle portion of driving means 10 is slidably guided in a slide bearing 28, which is rigidly fixed to the wall of shaft 4, and in a bayonet cylinder 30. The bayonet cylinder 30 can be longitudinally moved inside the shaft 4 within a limited range. A compression spring 32 abuts against the distal end face of the slide bearing 28 and against the proximal end face of the bayonet cylinder 30, thus forcing the bayonet cylinder in distal direction.

When the button 14 is pressed the actuating member 12 is moved in distal direction against the force exerted by compression spring 22. The actual position of the bayonet cylinder 30 does not affect this movement. The bayonet cylinder 30, the compression spring 32 and a bayonet slot 34 provided at the distal end of sleeve 6 form parts of a bayonet joint which is used to connect the magazine member 40.

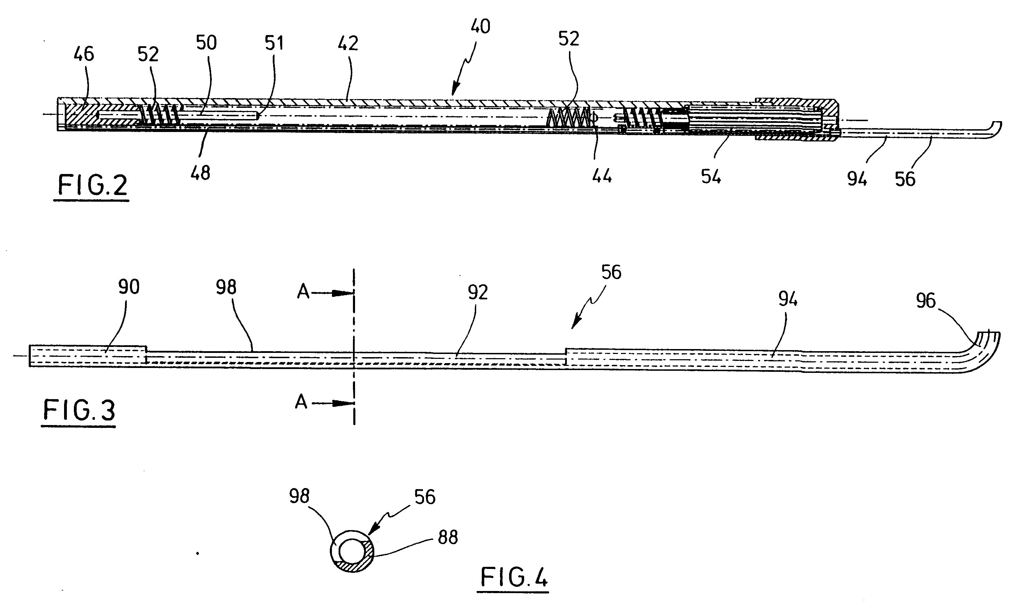

Figure 2 is an overall sectional view of the magazine member 40. At its housing 42 a transversely extending bayonet pin 44 is fixed. In order to mount the magazine member 40 at the distal end of shaft 4 the housing 42 is inserted into the sleeve 6. As usual with a bayonet joint, the bayonet pin 44 is guided in the bayonet slot 34, whereby the compression spring 32 is compressed via the bayonet cylinder 30.

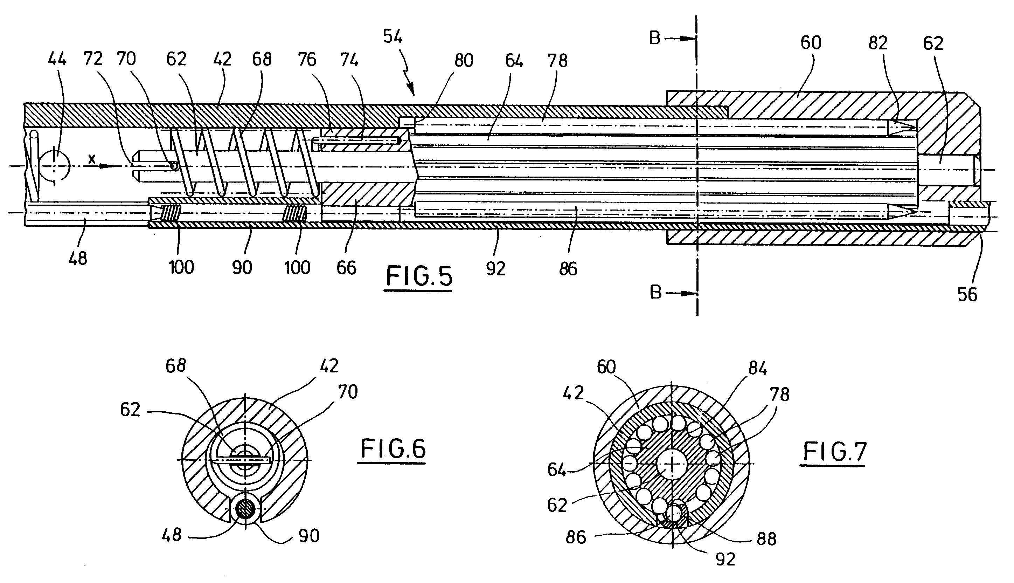

Inside the cylindrical housing 42 a guide cylinder 46 is slidably mounted. Close to its periphery a thin cylindrical rod 48 is attached to it which extends longitudinally in distal direction, see also Figure 5. The guide cylinder 46 and the rod 48 form the distal parts of driving means 10. A stop pin 50 is fixed to the guide cylinder 46 and extends in distal direction along the longitudinal axis L-L of the magazine member 40. A compression spring 52, which is only partially shown in Figure 2, abuts against the distal face of the guide cylinder 46 and against the bayonet pin 44 which also traverses the interior of housing 42. When the button 14 is actuated the distal end 27 of the middle portion 26 of driving means 10 moves the guide cylinder 46 in distal direction, against the additional force exerted by compression spring 52. The distance between the distal end 51 of the stop pin 50 and the bayonet pin 44 defines the range of movement. When the button 14 is released the proximal portion and the middle portion 26 of driving means 10 are retracted via the compression spring 22, while the guide cylinder 46 and the rod 48 are shifted in proximal direction by means of the action of compression spring 52.

In its distal region the magazine member 40 comprises a fastener magazine 54 and a fastener tube 56 which forms a longitudinally extending protrusion at the distal end, see also Figures 3 and 4.

Figure 5 shows an enlarged longitudinal section of the fastener magazine 54. A housing cap 60 is rigidly attached to the distal end of housing 42. A bearing pin 62 which extends along the longitudinal axis L-L of the magazine member 40 is fixed to the housing cap 60, e.g. by inserting into a hole and by welding. The bearing pin 62 defines the axis of a drum 64 which is rotatably mounted on the bearing pin 62. Preferably the drum is made of a plastic material, e.g. polycarbonate, whereas the bearing pin 62 and other parts of the magazine member 40 consist of a metal, e.g. stainless steel.

The drum 64 is biased for rotation in a given direction by means of a spring 68. To achieve this function, the proximal end 70 of spring 68 is held by a slot 72 provided at the proximal end of the bearing pin 62, see also Figure 6. The distal end 74 of spring 68 is inserted into a hole 76 provided in the proximal portion 66 of drum 64. Before assembling the magazine member 40 the spring 68 has to be tensioned by turning one end while the other end is fixed. Although the spring 68 looks like a compression spring, it acts as a torsion spring.

Fasteners 78 which are to be applied by the surgical instrument according to the invention are located along the lateral area of the drum 64. Before application the fasteners 78 are straight; they have flat end sides 80 and suitable points 82 to aid tissue penetration on the distal side. As shown in Figure 7, the fasteners 78 rest in trough-like recesses 84 provided on the lateral area of the drum 64 and they fill the space between the outer periphery of drum 64 and the inner surface of the distal region of housing 42.

In the preferred embodiment the fastener 86 to be applied during the next actuation of button 14 is already located in the fastener tube 56, see Figure 5 and Figure 7. The design of the fastener tube 56 is shown in Figure 3 and in Figure 4. The fastener tube does not only protrude from the distal end of the fastener magazine 54, but it also extends inside the magazine member 40. A proximal portion 90 of the fastener tube 56 is located adjacent to the spring 68, see also Figure 5. In a magazine portion 92 extending throughout the region of the fastener magazine 54 a longitudinal opening 98 is provided in the wall of the fastener tube 56, see also Figure 4. The fastener 86 to be applied has been moved into the fastener tube 56 through this longitudinal opening 98. The part of the wall of the fastener tube 56 opposing the longitudinal opening 98 forms a stop 88 extending in longitudinal direction. The fastener to be applied is pressed against this stop 88, thus preventing the drum 64 from an undesired rotation.

A protruding portion 94 of the fastener tube 56 projects from the distal end of the housing cap 60. The distal end portion 96 of the fastener tube 56 is curved and may be shaped in different ways in order to achieve a given curvature of the applied fastener. For example, the distal end of fastener tube 56 may be slightly offset laterally from the longitunal axis of the protruding portion 94. This offset causes the pointed end of the fastener to be offset or laterally spaced from the trailing end as the fastener is placed. For details see U.S. Patent No. 4,595,007.

The distal end of the driving means 10 is preferably formed as a helical spring 100 which is fixed to the distal end of rod 48. By means of the helical spring 100 the driving means 10 is guided inside the proximal portion 90 of fastener tube 56 when the driving means 10 is in its retracted position. When actuated, the rod 48 and the helical spring 100 are moved in distal direction until the distal end 51 of stop pin 50 touches the bayonet pin 44. In this position the distal end of the helical spring 100 preferably lies at the opening at the distal end of fastener tube 56. As the helical spring 100 is flexible, it is able to adjust to the curvature of the distal end portion 96 of fastener tube 56.

The surgical instrument for applying fasteners works in the following way:

Before an operation begins, a fresh magazine member 40, for example a disposable product, which is sterile, filled with suitable fasteners 78 and having a loaded spring 68, is inserted into the sleeve 6 and fixed by means of the bayonet joint, as described above. When the surgeon wants to apply a fastener he presses the button 14 thus actuating the driving means 10. This causes the rod 48 and the helical spring 100 to be shifted in distal direction. The distal end of the helical spring 100 engages to the end side 80 of the fastener 86 to be applied and pushes it into the protruding portion 94 of fastener tube 56. Inside the fastener magazine 54, the rod 48 now takes the position of the fastener 86 to be applied, which means that rotational movement of the drum 64 is still inhibited. While the fastener 86 to be applied is moved through the curved distal end portion 96 of fastener tube 56, it is bent. The leading part of the fastener 86 which has already left the fastener tube 56 keeps its curved shape while it penetrates tissue. In this way, annular or helical fastener structures for joining tissue together can be formed, as described in U.S. Patent No. 4,595,007. Finally, when the distal end of the helical spring 100 is at the distal end of fastener tube 56 (or in the neighbourhood of the distal end of fastener tube 56), the fastener 86 has been completely pushed out of the fastener tube 56.

When the surgeon releases the actuating member 12, the driving means 10 is moved back to its original position via the compression spring 22 and the compression spring 52. This means that the rod 48 and the helical spring 100 are shifted in proximal direction until they reach their final position as shown in Figure 5. As soon as the helical spring 100 has left the region of the fastener magazine 54 the movement of drum 64 is no longer blocked. Driven by the spring 68, the drum 64 rotates in a counterclockwise sense (see Figure 7). The remaining fasteners 78 resting in the recesses 84 are moved together with the drum 64 until the next fastener 78 enters through the longitudinal opening 98 into the magazine portion 92 of fastener tube 56 and abuts against the stop 88, thus inhibiting further movement of the drum 64.

After finishing the operation, the magazine member 40 can be removed from sleeve 6 and can be disposed of. The other parts of the surgical instrument have to be sterilized before used again.

## Claims

1. Surgical instrument for applying fasteners, especially for endoscopic purposes, comprising a handle (2), a shaft (4) attached to said handle (2), a driving means (10) being longitudinally shiftably guided in said shaft (4) and being coupled at its proximal end to an actuating member (12, 14) which is accessible from said handle (2), a fastener magazine (54) located in the distal region of said shaft (4) and being tensioned by a spring for fasteners (78) which are straight before application, and a fastener tube (56) extending from said fastener magazine (54), the distal end portion (96) of said fastener tube (56) being curved, the fastener (86) to be applied being shiftable by engagement of said driving means (10, 48, 100) via its end side out of said fastener magazine (54) into said fastener tube (56) and being pushable out of the distal end portion (96) of said fastener tube (56), **characterized** in that said fastener magazine (54) comprises a rotatable drum (64) being biased by said spring (68) into a rotational direction, said fasteners (78) being arranged essentially parallel to the rotational axis (L-L) in the peripheral region of said drum (64).

2. Surgical instrument for applying fasteners according to claim 1, **characterized** in that at its longitudinal side directed to the rotational direction of said drum (64) the fastener (86) to be applied abuts against a stop (88) which does not participate in the rotational movement of said drum (64).

3. Surgical instrument for applying fasteners according to claim 2, **characterized** in that said fastener tube (56) extends in proximal direction into said fastener magazine (54) and is provided there with a longitudinal opening (98) through which the fastener (86) to be applied is movable into said fastener tube (56), the wall portion of said fastener tube (56) opposing said longitudinal opening (98) forming said stop (88).

4. Surgical instrument for applying fasteners according to one of claims 1 to 3, **characterized** in that said drum (64) comprises trough-like recesses (84) on its lateral area for accommodating said fasteners (78).

5. Surgical instrument for applying fasteners according to one of claims 1 to 4, **characterized** in that in the distal region of said shaft (4) said driving means (10) comprises an essentially cylindrical rod (48) which is laterally transposed with respect to the longitudinal axis (L-L) of said shaft (4), the diameter of said rod (48) being slightly smaller than the interior dimensions of said fastener tube (56).

6. Surgical instrument for applying fasteners according to one of claims 1 to 5, **characterized** in that the distal end portion (100) of said driving means (10) is flexible.

7. Surgical instrument for applying fasteners according to claims 5 and 6, **characterized** in that at the distal end of said rod (48) a helical spring (100) extending longitudinally with respect to said rod (48) is fixed, the outer diameter of said helical spring (100) being smaller than the interior dimensions of said fastener tube (56).

8. Surgical instrument for applying fasteners according to one of claims 1 to 7, **characterized** in that said fastener magazine (54) including said fastener tube (56) extending therefrom is detachably fixed to said shaft (4).

9. Surgical instrument for applying fasteners according to claim 8, **characterized** in that the distal portion (46, 48, 100) of said driving means (10), said fastener magazine (54) and said fastener tube (56) are included in a magazine member (40) which is detachably fixed to said shaft (4).

10. Surgical instrument for applying fasteners according to claim 9, **characterized** in that a bayonet joint (6, 30, 32, 34, 44) is provided for fixing, said bayonet joint (6, 30, 32, 34, 44) permitting the coupling of the distal portion (46, 48, 100) of said driving means (10) to the proximal portion of said driving means (10).

11. Surgical instrument for applying fasteners according to one of claims 1 to 7, **characterized** in that it is designed to be fully disposable.

## Patentansprüche

1. Chirurgisches Klammersetzgerät, insbesondere für endoskopische Zwecke, mit einem Griff (2), mit einem am Griff (2) angebrachten Schaft (4), in dem längsverschiebbar ein Übertragungsteil (10) geführt ist, das an seinem proximalen Ende mit einem vom Griff (2) aus zugänglichen Betätigungsteil (12, 14) gekoppelt ist, mit einem im distalen Bereich des Schafts (4) angeordneten, unter Spannung einer Feder stehenden Klammermagazin (54) für vor ihrer Applizierung gerade Klammern (78), und mit einer von dem Klammermagazin (54) ausgehenden Klammerröhre (56), deren distaler Endbereich (96) gebogen ist, wobei die zu setzende Klammer (86) durch stirnseitigen Angriff des Übertragungsteils (10, 48, 100) aus dem Klammermagazin (54) in die Klammerröhre (56) schiebbar und aus deren distalem Endbereich (96) ausstoßbar ist, **dadurch gekennzeichnet**, daß das Klammermagazin (54) eine drehbare, durch die Feder (68) in Drehrichtung vorgespannte Trommel (64) aufweist, in deren peripherem Bereich die Klammern (78) im wesentlichen parallel zur Drehachse (L-L) angeordnet sind.

2. Chirurgisches Klammersetzgerät nach Anspruch 1, **dadurch gekennzeichnet**, daß die zu setzende Klammer (86) an ihrem in Drehrichtung der Trommel (64) weisenden Längsbereich an einem an der Drehbewegung der Trommel (64) nicht teilnehmenden Anschlag (88) anliegt.

3. Chirurgisches Klammersetzgerät nach Anspruch 2, **dadurch gekennzeichnet**, daß sich die Klammerröhre (56) in proximaler Richtung in das Klammermagazin (54) hinein fortsetzt und dort mit einer Längsaussparung (98) versehen ist, durch die hindurch die zu setzende Klammer (86) in die Klammerröhre (56) hinein bewegbar ist, wobei die der Längsaussparung (98) gegenüberliegende Wandung der Klammerröhre (56) den Anschlag (88) bildet.

4. Chirurgisches Klammersetzgerät nach einem der Ansprüche 1 bis 3, **dadurch gekennzeichnet**, daß die Trommel (64) auf ihrer Mantelfläche muldenartige Vertiefungen (84) zur Aufnahme der Klammern (78) aufweist.

5. Chirurgisches Klammersetzgerät nach einem der Ansprüche 1 bis 4, **dadurch gekennzeichnet**, daß das Übertragungsteil (10) im distalen Bereich des Schafts (4) einen in bezug auf die Längsachse (L-L) des Schafts (4) versetzten, im wesentlichen zylindrischen Stab (48) aufweist, dessen Durchmesser geringfügig kleiner ist als die Innenmaße der Klammerröhre (56).

6. Chirurgisches Klammersetzgerät nach einem der Ansprüche 1 bis 5, **dadurch gekennzeichnet**, daß der distale Endbereich (100) des Übertragungsteils (10) flexibel ist.

7. Chirurgisches Klammersetzgerät nach Anspruch 5 und 6, **dadurch gekennzeichnet**, daß am distalen Ende des Stabs (48) eine sich in Längsrichtung des Stabs (48) erstreckende Schraubenfeder (100) befestigt ist, deren Außendurchmesser kleiner ist als die Innenmaße der Klammerröhre (56).

8. Chirurgisches Klammersetzgerät nach einem der Ansprüche 1 bis 7, **dadurch gekennzeichnet**, daß das Klammermagazin (54) mit der davon ausgehenden Klammerröhre (56) abnehmbar an dem Schaft (4) befestigt ist.

9. Chirurgisches Klammersetzgerät nach Anspruch 8, **dadurch gekennzeichnet**, daß der distale Bereich (46, 48, 100) des Übertragungsteils (10), das Klammermagazin (54) sowie die Klammerröhre (56) in einem Magazinteil (40) enthalten sind, das abnehmbar an dem Schaft (4) befestigt ist.

10. Chirurgisches Klammersetzgerät nach Anspruch 9, **dadurch gekennzeichnet**, daß zur Befestigung ein die Ankopplung des distalen Bereichs (46, 48, 100) des Übertragungsteils (10) an den proximalen Bereich des Übertragungsteils (10) ermöglichender Bajonettverschluß (6, 30, 32, 34, 44) vorgesehen ist.

11. Chirurgisches Klammersetzgerät nach einem der Ansprüche 1 bis 7, **dadurch gekennzeichnet**, daß es als Einwegartikel konzipiert ist.

## Revendications

1. Instrument chirurgical pour l'application d'agrafes, en particulier à des fins endoscopiques, comprenant une poignée (2), un arbre (4) fixé a ladite poignée (2), un moyen d'entraînement (10) qui est guidé de manière à pouvoir se déplacer longitudinalement dans ledit arbre (4) et qui est accouplé, à son extrémité proximale, a un élément d'actionnement (12, 14) qui est accessible depuis ladite poignée (2), un magasin à agrafes (54) situé dans la région distale dudit arbre (4) et qui est placé sous tension par un ressort pour les agrafes (78), qui sont droites avant application, et un tube d'agrafes (56) s'étendant dudit magasin d'agrafes (54), la partie d'extrémité distale (96) dudit tube d'agrafes (56) étant incurvée, l'agrafe (86) a appliquer pouvant être déplacée par engagement desdits moyens d'entraînement (10, 48, 100) par son côté d'extrémité hors dudit magasin d'agrafes (54) dans ledit tube d'agrafes (56) et pouvant être expulsée de la partie d'extrémité distale (96) du tube à agrafes (56), caractérisé en ce que ledit magasin à agrafes (54) comprend un tambour rotatif (64) qui est sollicité par ledit ressort (68) dans un sens de rotation, lesdites agrafes (78) étant agencées essentiellement parallèlement a l'axe de rotation (L-L) dans la région périphérique dudit tambour (64).

2. Instrument chirurgical pour appliquer des agrafes selon la revendication 1, caractérisé en ce que, sur son côté longitudinal dirigé dans le sens de rotation dudit tambour (64), l'agrafe (86) à appliquer s'appuie contre un arrêt (88) qui ne participe pas au mouvement de rotation dudit tambour (64).

3. Instrument chirurgical pour appliquer des agrafes selon la revendication 2, caractérisé en ce que ledit tube a agrafes (56) s'étend dans la direction proximale dans ledit magasin d'agrafes (54) et y est pourvu d'une ouverture longitudinale (98) à travers laquelle l'agrafe (86) à appliquer peut se déplacer dans ledit tube à agrafes (56), la partie de paroi dudit tube à agrafes (56) étant opposée à ladite ouverture longitudinale (98) formant ledit arrêt (88).

4. Instrument chirurgical pour appliquer des agrafes selon l'une quelconque des revendications 1 à 3, caractérisé en ce que ledit tambour (64) comprend des évidements en forme d'auge (84) sur sa surface latérale pour recevoir lesdites agrafes (78).

5. Instrument chirurgical pour appliquer des agrafes selon l'une quelconque des revendications 1 à 4, caractérisé en ce que, dans la région distale dudit arbre (4), lesdits moyens d'entraînement (10) comprennent une tige essentiellement cylindrique (48) qui est transposée latéralement par rapport à l'axe longitudinal (L-L) dudit arbre (4), le diamètre de ladite tige (48) étant légèrement moindre que les dimensions internes dudit tube à agrafes (56).

6. Instrument chirurgical pour appliquer des agrafes selon l'une quelconque des revendications 1 à 5, caractérisé en ce que la partie d'extrémité distale (100) desdits moyens d'entraînement (10) est flexible.

7. Instrument chirurgical pour appliquer des agrafes selon les revendications 5 et 6, caractérisé en ce que, à l'extrémité distale de ladite tige (48), un ressort hélicoïdal (100) s'étendant longitudinalement par rapport à ladite tige (48) est fixé, le diamètre externe dudit ressort hélicoïdal (100) étant plus petit que les dimensions internes dudit tube à agrafes (56).

8. Instrument chirurgical pour appliquer des agrafes selon l'une quelconque des revendications 1 à 7, caractérisé en ce que ledit magasin à agrafes (54) comprenant ledit tube à agrafes (56) qui le prolonge, est fixé de manière détachable audit arbre (4).

9. Instrument chirurgical pour appliquer des agrafes selon la revendication 8, caractérisé en ce que la partie distale (46, 48, 100) desdits moyens d'entraînement (10), dudit magasin d'agrafes (54) et dudit tube à agrafes (56) sont inclus dans un élément de magasin (40) qui est fixé de manière détachable audit arbre (4).

10. Instrument chirurgical pour appliquer des agrafes selon la revendication 9, caractérisé en ce qu'un joint à baïonnette (6, 30, 32, 34, 44) est prévu pour la fixation, ledit joint à baïonnette (6, 30, 32, 34, 44) permettant d'accoupler la partie distale (46, 48, 100) desdits moyens d'entraînement (10) à la partie proximale desdits moyens d'entraînement (10).

11. Instrument chirurgical pour appliquer des agrafes selon l'une quelconque des revendications 1 à 7, caractérisé en ce qu'il est conçu pour être totalement jetable.
